# EUROPEAN PATENT APPLICATION

(11) **EP 1 752 169 A2**
(43) Date of publication of application: **14.02.2007**
(21) Application number: 06251834.5
(22) Date of filing: 31.03.2006
(51) Int. Cl.: A61L 29/00, A61M 25/10

(54) **Semi-compliant balloon for medical devices**

(30) Priority: 31.03.2005 US 95310
(71) Applicant: Cordis Neurovascular, Inc., Miami Lakes, Florida 33014 (US)
(72) Inventor: Pedroso, Pedro D., Florida 33015 (US); Slazas, Robert, Miami Florida 33176 (US)
(74) Representative: Belcher, Simon James

(57) **Abstract**

A balloon assembly for a medical device includes an inflatable body formed of a semi-compliant material defining at least one inflation chamber which is adapted for inflation and deflation. The semi-compliant material is made of a blend of a first formulation which comprises an organofluorine polymer in sufficient concentration to provide lubricity without the presence of a lubricating surface layer of different formulation and a second formulation which is substantially-fluorine free.

## Description

The invention relates generally to the field of intravascular catheters, and more particularly to a balloon for use with a catheter formed from a fluorine-containing polymer blend.

Balloons mounted on the distal ends of catheters are widely used in medical treatment. The balloon may be used to widen a vessel into which the catheter is inserted or to force open a blocked vessel. Balloons, particularly balloons for the vascular system generally require low friction surfaces in their exterior surfaces, so that the balloon may be easily advanced. To accomplish this, lubricating coatings have been applied to balloons. However, such coatings can wear away and thus lose their effectiveness.

While angioplasty balloons are considered to be inelastic relative to balloons used in most other applications, there is in the art a general classification of such balloons based on their expandability or "compliance" relative to one another. "Non-compliant" balloons are the least elastic, increasing in diameter the least as the balloon is pressurized to an inflation pressure. "Semi-compliant" balloons have somewhat greater extensibility over the pressurization range. "Compliant" balloons are still more extensible.

One suggested solution to the problem of lubricity for a balloon includes Narisco, Jr., US-5456661, in which a wall of balloon body may be made of "a fluoropolymer resin". However, such resin may be disadvantageous, in that the balloon lacks semi-compliant physical properties which may be more desirable for a balloon, although it does provide a low friction surface.

By this invention, a balloon of reduced cost may be provided, containing less fluorine than in the balloon disclosed in US-5456661 and exhibiting desired, low friction surfaces, particularly at critical areas of the balloon where the low friction is most needed, while remaining semi-compliant. Furthermore, by this invention, the fluorine containing material from which the balloon can be made may have a wide range of adjustability of physical properties so that, along with the low friction characteristic, the balloon may have optimum, desirable, physical characteristics.

While a balloon for use with a catheter is specifically disclosed herein, it is contemplated that other medical devices may be at least partly made from one of the desirable formulations of this invention, to achieve a low surface tension, coupled with a desirable variability of other physical parameters, as may be provided by this invention.

The illustrative embodiment of the present invention relates to a balloon assembly for a medical device including an inflatable body formed of a semi-compliant material defining at least one inflation chamber which is adapted for inflation and deflation. The semi-compliant material is made of a blend of a first formulation which comprises an organofluorine polymer in sufficient concentration to provide lubricity without the presence of a lubricating surface layer of different formulation and a second formulation which is substantially-fluorine free.

Further in accordance with this invention, a balloon may comprise an inflatable body that may be made of a blend that comprises:
(a) a relatively soft polymer, for example an elastomer;
(b) a polymer that is relatively stiff, such as a stiff nylon, compared with the relatively soft ingredient (a) above; and
(c) a fluorine-containing polymer, in some embodiments no more than 10 weight percent.

The invention provides a balloon for use with medical devices, such as catheters, that has semi-compliant physical properties and provides a low friction surface.

A more detailed explanation of the invention is provided in the following description and claims and is illustrated in the accompanying drawings.

The balloon cather of the present invention may be used in a method for performing a medical procedure, the method comprising: inserting into a tissue of a patient an inflatable body formed of a semi-compliant material defining at least one inflation chamber which is adapted for inflation and deflation, the semi-compliant material being made of a blend of a first formulation which comprises an organofluorine polymer in sufficient concentration to provide lubricity without the presence of a lubricating surface layer of different formulation and a second formulation which is substantially-fluorine free; and inflating the at least one inflation chamber.

Embodiments of the invention will now be described by way of example with reference to the accompanying drawings, in which:
FIG. 1 is a perspective view of a vascular balloon catheter in accordance with this invention; and
Fig. 2 is an enlarged, longitudinal sectional view of the tip area of the catheter of Fig. 1.

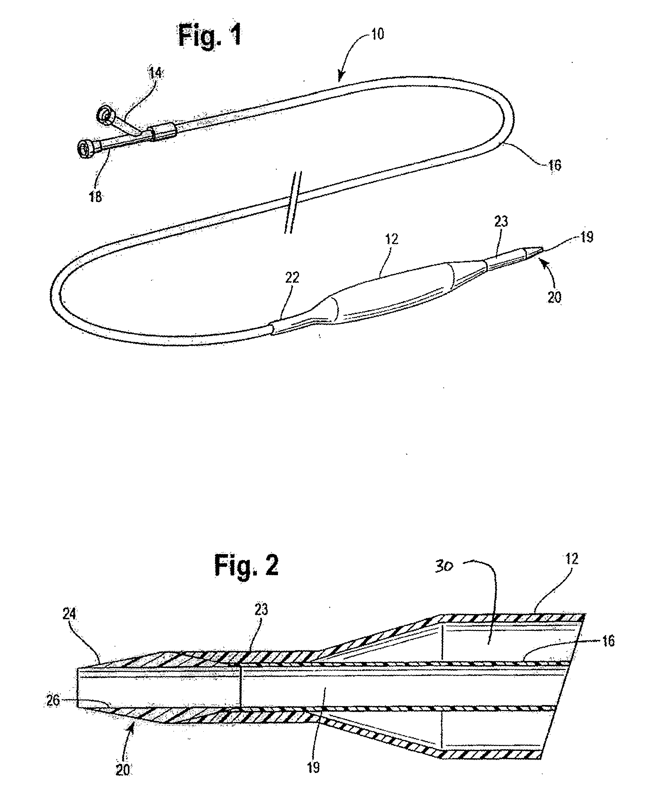

Referring to the drawings, Figs. 1 and 2 show a catheter 10 which may be used for angioplasty or the like, having an angioplasty balloon 12, which is connected to proximal inflation port 14 by a conventional inflation lumen within a catheter sheath or body 16. Guidewire port 18 connects through another conventional lumen 19, which extends through the entire length of catheter 10, extending also through the distal end of the catheter at catheter tip 20.

Balloon 12 may be connected to catheter sheath or body 16 in the conventional manner, by sealing at both ends thereof 22, 23. For purposes of disclosure, balloon assembly 12, 22, 23, is deemed to be a part of tubular catheter body 16.

Tubular catheter body 16 and balloon assembly 12, 22, 23, may be formed from a blend of polymers that form a semi-compliant material, with balloon 12 adapted for inflation from a folded configuration to an expanded configuration and deflation back to a folded configuration. The semi-compliant material may be made of a blend of a first formulation which comprises an organofluorine polymer in sufficient concentration to provide lubricity without the presence of a lubricating surface layer of different formulation and a second formulation which is substantially-fluorine free. Balloon assembly 12, 22, 23 may also be made of a formulation which comprises an intimate blend or mixture of ingredients (a), (b) and (c) as described above, having sufficient fluorine content (typically less than 10 wt. percent) to provide an inherently lubricating, low friction balloon surface. Inflation port 14 communicates with inflation/deflation chamber 30. Guidewire lumen 19, which is a dedicated channel for a guidewire (not shown), may also be used for providing fluid, such as a gas or liquid, from an external port to the treatment site.

As the catheter is advanced and retracted on the guidewire, and balloon 12 is likewise advanced and retracted against a blood vessel, during the angioplasty procedure or the like, the low friction characteristic of balloon 12 is retained. This friction reduction does not wear away, because it is not provided by a special, frictional coating, but is rather an inherent characteristic of the formulation of balloon 12.

The fluorine-containing polymer of the blend or mixture of the illustrative embodiment may comprise, for example, polymers and co-polymers of polyvinylidene fluoride (PVDF), polytetrafluoroethylene (PTFE), or fluoroelastomers, such as the known fluoroelastomers of hexafluoropropylene and vinylidene fluoride, which fluoroelastomers may optionally also contain PTFE. An examples of these elastomers are sold under the trademark Viton. Also, a fluorinated polymer processing additive may be used as ingredient (c), as well as Kynar, a PVDF product of Arkema. Also, non-fluorinated polymers carrying fluorinated polymer powders such as PTFE may be used. Generally, the amount of fluorine-containing polymer needed varies inversely with the hardness of the other ingredients (a), (b) present: softer materials may need more, and harder materials may need less fluorinated material for improved lubricity.

The relatively soft polymer of ingredient (a) may comprise elastomers such as poly(ether-nylon) block co-polymers, but, alternatively, relatively soft, elastomeric or non-elastomeric materials may be used such as polyethylene, polyvinyl chloride, polyesters, soft polyamides, polyurethane, or silicone rubber. Copolymers of the above may also be used.

The relatively stiff polymer of ingredient (b) may comprise nylon, (i.e. a polyamide), or polypropylene, or other known, stiff materials.

The respective ingredients (a), (b) and (c) may be selected to be compatible with each other, so that the resulting mixture is homogeneous to a desired degree, possessing a low surface friction despite the fact that the fluorine-containing polymer of ingredient (c) is present in only a minor proportion. As a result of the lesser amount of fluorine-containing polymer in the blend or mixture, balloons of the present invention have somewhat greater extensibility over the pressurization range and are therefore semi-compliant.

The fluorine present in the polymer is typically substantially carbon-bonded fluorine.

In some embodiments, the blend comprises a mixture of:
(a) a relatively soft polymer, for example an elastomer;
(b) a polymer that is relatively stiff, such as a stiff nylon, compared with the relatively soft ingredient (a) above; and
(c) a fluorine-containing polymer, in some embodiments no more than 10 weight percent.

In other embodiments, the blend comprises a mixture of:
(a) from about 55 to 99.9 weight percent of a relatively soft polymer;
(b) from about 0 to 30 weight percent of a compatible polymer that is relatively stiff, compared with ingredient (a) above; and
(c) from about 0.1 to 15 weight percent of a fluorine-containing polymer.

In yet other embodiments, the blend comprises a mixture of
(a) From about 70 to 94 weight percent of a relatively soft polymer;
(b) From about 5 to 25 weight percent of a compatible polymer that is relatively stiff, compared with ingredient (a) above; and
(c) From about 0.2 to 5 weight percent of a fluorine-containing polymer.

It can be seen that by adjustment of the amounts of particularly ingredients (a) and (b), a range of tensile strengths and overall softness of the formulation, and the balloons resulting therefrom, can be achieved, to tailor make desired flexibilities and softness in the balloons of this invention, typically having properties roughly intermediate between the relatively soft polymer of ingredient (a) and the relatively stiff polymer of ingredient (b). In some embodiments, the relatively soft polymer is elastomeric. Ingredient (c) then provides inherent lubricity at a relatively low total fluorine concentration in the whole formulation, for example - about 0.1 to 5 weight percent of fluorine.

In some preferred formulations, it has been deemed surprising that relatively homogeneous mixtures of the three ingredients (a), (b) and (c) can be made without significant phase incompatibility.

## Claims

1. A balloon for a medical device, comprising:
an inflatable body formed of a semi-compliant material defining at least one inflation chamber which is adapted for inflation and deflation, the semi-compliant material being made of a blend of a first formulation which comprises an organofluorine polymer in sufficient concentration to provide lubricity without the presence of a lubricating surface layer of different formulation and a second formulation which is substantially-fluorine free.

2. The balloon according to claim 1, wherein the total fluorine content is 0.1 to 5 weight percent.

3. The balloon according to claim 1, wherein the first formulation further comprises from about 0.1 to 15 weight percent of a fluorine-containing polymer.

4. The balloon according to claim 1, wherein the second formulation further comprises:
(a) from about 55 to 99.9 weight percent of a relatively soft polymer; and
(b) from about 0 to 30 weight percent of a compatible polymer that is relatively stiff, compared with ingredient (a) above.

5. The balloon according to claim 1, wherein the first formulation further comprises from about 0.2 to 5 weight percent of a fluorine-containing polymer.

6. The balloon according to claim 1, wherein the second formulation further comprises:
(a) from about 70 to 94 weight percent of a relatively soft polymer; and
(b) from about 5 to 25 weight percent of a compatible polymer that is relatively stiff, compared with ingredient (a) above.

7. The balloon according to claim 3 or 5, wherein the fluorine-containing polymer comprises polyvinylidene fluoride.

8. The balloon according to claim 4 or 6, wherein the relatively stiff polymer comprises a nylon.

9. The balloon according to claim 4 or 6, wherein the relatively soft polymer comprises a poly(ether-nylon) block co-polymer.

10. The balloon according to claim 9, wherein the fluorine present in the polymer is substantially carbon-bonded fluorine.
